# EUROPEAN PATENT SPECIFICATION

(11) **EP 1 716 848 B1**
(45) Date of publication and mention of the grant of the patent: **25.04.2007**
(21) Application number: 05380082.7
(22) Date of filing: 25.04.2005
(51) Int. Cl.: A61K 9/20, A61K 31/445

(54) **Solid pharmaceutical composition containing a crystalline derivative of piperidine substituted in the 1,4 position**
Feste Arzneizubereitung enthaltend ein kristallines in 1,4-Stellungen substituiertes Piperidinderivat
Composition pharmaceutique solide contenant une forme crystalline de piperidine substituée en positions 1,4

(43) Date of publication of application: 02.11.2006
(73) Proprietor: LABORATORIOS ALTER, S.A., 28036 Madrid (ES)
(72) Inventor: Duro Fernández, Roberto, 28034 Madrid (ES); Nieto Mendoza, Maria del Carmen, 28005 Madrid (ES)
(74) Representative: Ungria Lopez, Javier

(56) References cited:
- EP-B- 0 614 362

## Description

### TECHNICAL FIELD OF THE INVENTION

The present invention belongs to the technical field of pharmaceutical compositions containing piperidine derivatives substituted in the 1,4 position as active ingredient. More specifically, it comes within the sector of solid compositions in which said derivative is present in crystalline form.

### STATE OF THE ART PRIOR TO THE INVENTION

The European patent application EP-A-0134124 describes piperidine derivatives substituted in the 1,4 position, of formula 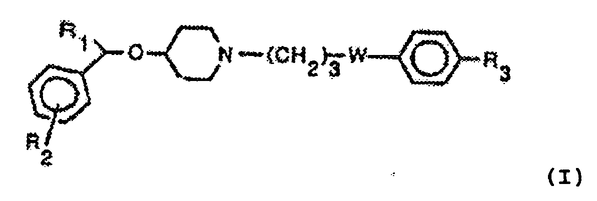 in which
- R1 is a thienyl group, a phenyl group, a phenyl group substituted with a halogen, an alcoxy group with 1 to 6 carbon atoms or an alkyl group with 1 to 6 carbon atoms.
- R2 is a halogen atom, a hydrogen atom, an alcoxy group with 1 to 6 carbon atoms or an alkyl group with 1 to 6 carbon atoms.
- R3 is a halogen atom, a hydrogen atom, an alcoxy group with 1 to 6 carbon atoms, an alkyl group with 1 to 6 carbon atoms, an alkylthio group with 1 to 6 carbon atoms, a cycloalkyl group with 5 or 6 carbon atoms, or a group of formula: 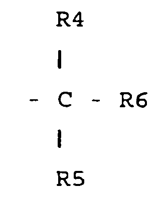
in which
R4 and R5 are, independently of each other, a hydrogen atom or an alkyl group with 1 to 6 carbon atoms.
R6 is a cycloalkyl group with 3 to 6 carbon atoms, or a hydroxymethyl, carboxy or alcoxycarbonyl group with 2 to 7 carbon atoms, and
W is a carbonyl or hydroxymethylene group.

These compounds, among which ebastine (2-diphenylmethoxy 1-[3-(4-terc.-butylbenzoyl)propyl]piperidine is to be found, displays H₁ antihistaminic activity. Nevertheless, when presented in solid form, it has low solubility in water resulting in a deficient bioavailability of this active principle.

The problem of bioavailability of these compounds, specifically ebastine, has sought to be solved, as described in European patent application EP-A-0614362, by micronising the ebastine in a way that is in itself conventional, thereby conferring the ebastine with a maximum granulometry of less than 200 µm, a mean granulometry by number of between 0.5 and 15 µm, in which preferably 90% by number of the particles have a granulometry of less than 25 µm and preferably less than 20 µm. This micronisation of ebastine corresponds to the general principle that, owing to the reduction in particle size, the micronisation of any solid pharmaceutical active principle implies an increase in the effective surface area of the solid particles of the drug, it entails an increase in the rate of dissolution and the consequent improvement in bioavailability.

Although the micronised ebastine of European patent application EP-A-0614362 seems to solve the problem of bioavailability, micronisation of the crystals of ebastine displays the drawbacks of being an additional stage in the manufacture of ebastine and of constituting an aggressive treatment that can damage the crystal structure of the ebastine, in addition to implying the formation of very small size powder particles, generally electrostatically charged, which hinder the processing and handling of the ebastine thus micronised when it comes to manufacturing tablets.

It was therefore a desirable objective to find a way of obtaining oral solid formulations that would enable the micronisation to be dispensed with, and which at the same time would offer a good solubility and, therefore, bioavailability of compounds of formula I and, especially, of ebastine.

### DESCRIPTION OF THE INVENTION

The present invention permits the objective stated above to be achieved by means of a solid pharmaceutical composition which comprises at least a first and a second excipient mixed with the crystalline active principle of formula (I) specified above, or a pharmaceutically acceptable salt of that active principle, in particles with a maximum size of less than 500 µm, in which the first excipient is a non-ionic tensioactive agent.

The non-ionic tensioactive agent is preferably selected from among the group consisting of fatty esters of polyethoxylenated sorbitan, marketed under the name of POLYSORBATE 80, a particularly suitable tensioactive. Surprisingly, the increase in aqueous solubility of the active principle of formula (I) is only produced when the tensioactive present in the composition is a non-ionic tensioactive but not when it is an anionic or amphoteric tensioactive.

The presence of the non-ionic tensioactive permits a suitable solubility to be achieved for piperidine derivatives substituted in the 1,4 position of formula (I) without the need to subject those derivatives to micronisation.

The composition can contain from 0.1 to 10% by weight of the tensioactive agent and, in a practical embodiment of the invention, the compound comprises 0.5 to 2% by weight of the tensioactive agent.

The composition can contain between 1 and 25% by weight of the active principle, preferably with a mean particle size by volume of between 15 and 100 µm and more preferably between 16 and 80 µm. In one embodiment, the compound comprises between 8 and 12% by weight of the active principle.

The active principle can display a granulometry in which 90% of the particles have a particle size less than 225 µm, and preferably less than 150 µm.

In a preferred embodiment of the invention, the active principle is ebastine.

In accordance with the invention, the second excipient can be a diluting agent in itself conventional, such as for example lactose, lactose monohydrate, mannitol, cellulose, inorganic phosphates, inorganic carbonates and combinations thereof, a preferred second excipient being lactose monohydrate. The second excipient can be included in the composition in a proportion of between 10-90% by weight and, especially, between 50 and 70% by weight.

The composition can also comprise, in a manner in itself conventional, a binding agent, a disintegrating agent, a filling agent and/or a lubricating agent.

The binding agent can be a binder in itself conventional, selected from the group comprising polyvinylpyrrolidone, hydroxypropylmethylcellulose, alginic acid, sodium alginate, polymethacrylate, maltodextrine, liquid glucose, aluminium silicate, hydroxypropylcellulose, aluminium silicate, magnesium silicate, starch and combinations thereof, a preferred binding agent being polyvidone.

The disintegrating agent can be selected from among disintegrating agents in themselves conventional, for example, from the group comprising alginic acid, sodium carboxymethylcellulose, colloidal silicon dioxide, sodium croscarmellose, crospovidone, sodium alginate, cellulose in powder form, aluminium and magnesium silicate, pregelatinised starch and combinations thereof, a preferred disintegrating agent being pregelatinised starch. This disintegrating agent can be present in the composition in a proportion of between 5 and 75% by weight and, in one embodiment of the invention, between 5 and 15% by weight of the disintegrant.

The filling agent can be a filling agent in itself conventional, for example, suitable ones being cellulose, lactose, calcium acid phosphate, microcrystalline cellulose and combinations thereof, one of the preferred filling agents being microcrystalline cellulose. The filling agent can be present in the composition in a proportion of between 5 and 90% by weight.

The lubricating agent can also be in itself conventional, suitable lubricating agents being calcium stearates, magnesium stearates, zinc stearates, glyceryl monostearates, glyceryl palmitostearates, hydrogenated castor oil, hydrogenated vegetable oils, light mineral oils, polyethyleneglycol, sodium benzoates, sodium lauryl sulphate (which is also an anionic tensioactive), sodium stearyl fumarate, stearic acids and combinations thereof. In a preferred embodiment, the lubricating agent is selected from among the group consisting of agents which also have compression coadjuvant function, as might be magnesium stearate, calcium stearate, zinc stearate and combinations thereof. The lubricating agent can be present in the composition in a proportion of between 0.25 and 5.0% by weight and preferably between 0.25 and 2% by weight.

The composition of the invention is preferably a tablet composition, and more preferably a tablet composition that comprises the crystalline active principle, a diluent, a binding agent, a disintegrating agent, a tensioactive agent and a compression coadjuvant agent. The method for obtaining the composition of the invention, as defined above, comprises:
preparing a first mixture comprising the crystalline active principle and a diluting agent,
passing the first mixture through a sieve with hole sizes between 0.5 and 0.7 mm, in order to obtain a first sieved mixture,
adding a disintegrating agent to the first sieved mixture, and optionally a filling agent, in order to obtain a second mixture,
blending the second mixture with an aqueous solution of the non-ionic tensioactive agent and, optionally a binding agent, in order to obtain a blended mixture,
drying the blended mixture until a dry mixture is obtained,
passing the dry mixture through a sieve with hole sizes between 1.8 to 2.2 mm, in order to obtain a second sieved mixture,
mixing the second sieved mixture with a compression coadjuvant agent, such as for example a lubricating agent, in order to obtain a compressible mixture, and
compressing at least part of the compressible mixture in order to obtain at least one tablet.

The diluting agent, the disintegrating agent, the filling agent, the non-ionic tensioactive agent and the compression coadjuvant agent can be those specified above.

### EMBODIMENTS OF THE INVENTION

Below, certain aspects will be explained on the basis of some examples. Examples 1 to 4 are examples of compositions that refers to background art. Examples 5 and 6 are examples of compositions related to the present invention and example 7 is an example of a test that compares the compositions of background art and the compositions of the present invention, obtained in the examples 1 to 6.
**EXAMPLE 1**: 100 g of ebastine of mean particle size between 40 and 45 microns (measured by laser beam diffraction in a Malvern 2000 apparatus) were mixed with 620 g of lactose monohydrate as diluent, and the first mixture thus obtained was passed through a sieve with a hole size of 0.5 mm, in order to obtain a first sieved mixture.

This sieved mixture was mixed with 100 g of pregelatinised starch as disintegrating agent and 165 g of microcrystalline cellulose as filling agent in order to obtain a second mixture.

The second mixture was blended with water and 10 g of polyvidone as binding agent, in order to obtain a blended mixture without any tensioactive agent which was dried and passed through a sieve with a hole size of 2 mm, in order to obtain a second sieved mixture. The second sieved mixture was mixed with 5 g of magnesium stearate as compression coadjuvant, obtaining a compressible mixture from which tablets were obtained, each one with a weight of 100 mg, by means of compression in moulds.

Each of the tablets had the following composition:

**Table 1**

| **Component** | **Content** |
|---|---|
| Ebastine | 10.00 mg |
| Lactose monohydrate | 62.00 mg |
| Microcrystalline cellulose | 16.50 mg |
| Pregelatinised starch | 10.00 mg |
| Polyvidone | 1.00 mg |
| Polysorbate 80 | --------------- |
| Sodium laurylsulphate | --------------- |
| Lecithin | --------------- |
| Sodium docusate | --------------- |
| Magnesium stearate | 0.50 mg |

**EXAMPLE 2:** 100 g of ebastine of mean particle size between 40 and 45 microns (measured by laser beam diffraction in a Malvern 2000 apparatus) were mixed with 620 g of lactose monohydrate as diluent, and the first mixture thus obtained was passed through a sieve with a hole size of 0.5 mm, in order to obtain a first sieved mixture.

This sieved mixture was mixed with 100 g of pregelatinised starch as disintegrating agent and 65 g of microcrystalline cellulose as filling agent in order to obtain a second mixture.

The second mixture was blended with 10 g of sodium laurylsulphate, an anionic tensioactive agent, dissolved in water and 10 g of polyvidone as binding agent, in order to obtain a blended mixture which was dried and passed through a sieve with a hole size of 2 mm, in order to obtain a second sieved mixture. The second sieved mixture was mixed with 5 g of magnesium stearate as compression coadjuvant, obtaining a compressible mixture from which tablets were obtained, each one with a weight of 100 mg, by means of compression in moulds.

Each of the tablets had the following composition:

**Table 2**

| **Component** | **Content** |
|---|---|
| Ebastine | 10.00 mg |
| Lactose monohydrate | 62.00 mg |
| Microcrystalline cellulose | 6.50 mg |
| Pregelatinised starch | 10.00 mg |
| Polyvidone | 1.00 mg |
| Polysorbate 80 | --------------- |
| Sodium laurylsulphate | 10.00 mg |
| Lecithin | --------------- |
| Sodium docusate | --------------- |
| Magnesium stearate | 0.50 mg |

**EXAMPLE 3:** 100 g of ebastine of mean particle size between 40 and 45 microns (measured by laser beam diffraction in a Malvern 2000 apparatus) were mixed with 620 g of lactose monohydrate as diluent, and the first mixture thus obtained was passed through a sieve with a hole size of 0.5 mm, in order to obtain a first sieved mixture.

This sieved mixture was mixed with 100 g of pregelatinised starch as disintegrating agent and 135 g of microcrystalline cellulose as filling agent in order to obtain a second mixture.

The second mixture was blended with 30 g of lecithin, an amphoteric tensioactive agent, dissolved in water and 10 g of polyvidone as binding agent, in order to obtain a blended mixture which was dried and passed through a sieve with a hole size of 2 mm, in order to obtain a second sieved mixture. The second sieved mixture was mixed with 5 g of magnesium stearate as compression coadjuvant, obtaining a compressible mixture from which tablets were obtained, each one with a weight of 100 mg, by means of compression in a compressing machine.

Each of the tablets had the following composition:

**Table 3**

| **Component** | **Content** |
|---|---|
| Ebastine | 10.00 mg |
| Lactose monohydrate | 62.00 mg |
| Microcrystalline cellulose | 13.50 mg |
| Pregelatinised starch | 10.00 mg |
| Polyvidone | 1.00 mg |
| Polysorbate 80 | --------------- |
| Sodium laurylsulphate | --------------- |
| Lecithin | 3.00 |
| Sodium docusate | --------------- |
| Magnesium stearate | 0.50 mg |

**EXAMPLE 4:** 100 g of ebastine of mean particle size between 40 and 45 microns (measured by laser beam diffraction in a Malvern 2000 apparatus) were mixed with 620 g of lactose monohydrate as diluent, and the first mixture thus obtained was passed through a sieve with a hole size of 0.5 mm, in order to obtain a first sieved mixture.

This sieved mixture was mixed with 100 g of pregelatinised starch as disintegrating agent and 155 g of microcrystalline cellulose as filling agent in order to obtain a second mixture.

The second mixture was blended with 10 g of sodium docusate, an anionic tensioactive agent, dissolved in water and 10 g of polyvidone as binding agent, in order to obtain a blended mixture which was dried and passed through a sieve with a hole size of 2 mm, in order to obtain a second sieved mixture. The second sieved mixture was mixed with 5 g of magnesium stearate as compression coadjuvant, obtaining a compressible mixture from which tablets were obtained, each one with a weight of 100 mg, by means of compression in moulds.

Each of the tablets had the following composition:

**Table 4**

| **Component** | **Content** |
|---|---|
| Ebastine | 10.00 mg |
| Lactose monohydrate | 62.00 mg |
| Microcrystalline cellulose | 15.50 mg |
| Pregelatinised starch | 10.00 mg |
| Polyvidone | 1.00 mg |
| Polysorbate 80 | --------------- |
| Sodium laurylsulphate | --------------- |
| Lecithin | --------------- |
| Sodium docusate | 1.00 mg |
| Magnesium stearate | 0.50 mg |

**EXAMPLE 5:** 100 g of ebastine of mean particle size between 40 and 45 microns (measured by laser beam diffraction in a Malvern 2000 apparatus) were mixed with 620 g of lactose monohydrate as diluent, and the first mixture thus obtained was passed through a sieve with a hole size of 0.5 mm, in order to obtain a first sieved mixture.

This sieved mixture was mixed with 100 g of pregelatinised starch as disintegrating agent and 155 g of microcrystalline cellulose as filling agent in order to obtain a second mixture.

The second mixture was blended with 10 g of POLYSORBATE 80, a non-ionic tensioactive agent, dissolved in water and 10 g of polyvidone as binding agent, in order to obtain a blended mixture which was dried and passed through a sieve with a hole size of 2 mm, in order to obtain a second sieved mixture. The second sieved mixture was mixed with 5 g of magnesium stearate as compression coadjuvant, obtaining a compressible mixture from which tablets were obtained, each one with a weight of 100 mg, by means of compression in moulds.

Each of the tablets had the following composition:

**Table 5**

| **Component** | **Content** |
|---|---|
| Ebastine | 10.00 mg |
| Lactose monohydrate | 62.00 mg |
| Microcrystalline cellulose | 15.50 mg |
| Pregelatinised starch | 10.00 mg |
| Polyvidone | 1.00 mg |
| Polysorbate 80 | 1.00 mg |
| Sodium laurylsulphate | --------------- |
| Lecithin | --------------- |
| Sodium docusate | --------------- |
| Magnesium stearate | 0.50 mg |

**EXAMPLE 6:** 100 g of ebastine of mean particle size between 40 and 45 microns (measured by laser beam diffraction in a Malvern 2000 apparatus) were mixed with 620 g of lactose monohydrate as diluent, and the first mixture thus obtained was passed through a sieve with a hole size of 0.5 mm, in order to obtain a first sieved mixture.

This sieved mixture was mixed with 100 g of pregelatinised starch as disintegrating agent and 155 g of microcrystalline cellulose as filling agent in order to obtain a second mixture.

The second mixture was blended with 10 g of POLYSORBATE 80, a non-ionic tensioactive agent, dissolved in water and 10 g of polyvidone as binding agent, in order to obtain a blended mixture which was dried and passed through a sieve with a hole size of 2 mm, in order to obtain a second sieved mixture. The second sieved mixture was mixed with 5 g of magnesium stearate as compression coadjuvant, obtaining a compressible mixture from which tablets were obtained, each one with a weight of 100 mg, by means of compression in moulds.

Each of the tablets had the following composition:

**Table 6**

| **Component** | **Content** |
|---|---|
| Ebastine | 10.00 mg |
| Lactose monohydrate | 62.00 mg |
| Microcrystalline cellulose | 11.50 mg |
| Pregelatinised starch | 10.00 mg |
| Polyvidone | 1.00 mg |
| Polysorbate 80 | 5.00 mg |
| Sodium laurylsulphate | --------------- |
| Lecithin | --------------- |
| Sodium docusate | --------------- |
| Magnesium stearate | 0.50 mg |

### EXAMPLE 7: DISSOLUTION TESTS

Six dissolution tests were conducted with six tablets of each of the batches obtained according to examples 1 to 6; along with an additional test with six tablets each of the original commercial drug of ebastine, Ebastel^{®} 10 mg.

The tests were conducted in a vane apparatus described in the European Pharmacopoeia, 5^{th} edition (2005) and in the USP XXVIII (2005), which contained a dissolution medium consisting of 1000 ml of 0.1 mol/l (N) hydrochloric acid. Each batch of tablets was tested separately, adding it to the dissolution medium in the vane apparatus. The speed of rotation of the vane was 100 rpm.

After 45 minutes, the percentage of ebastine dissolved in the dissolution medium was measured by spectrophotometry in the ultraviolet range with a wavelength of 257 in 1 cm dishes. The results of the seven dissolution tests are shown in the following table:

**Table 7**

| Example | Quantity of tensioactive per tablet | Dissolution % after 45 minutes |
|---|---|---|
| Example 1 | Without tensioactive | 17.1% |
| Example 2 | 10 mg sodium laurylsulphate | 13.4% |
| Example 3 | 3 mg lecithin | 19.7% |
| Example 4 | 1 mg sodium docusate | 30.7% |
| Example 5 | 1 mg polysorbate 80 | 66.4% |
| Example 6 | 5 mg polysorbate 80 | 67.3% |
| original | | |
| commercial | ------------------- | 66.9% |
| drug Ebastel^{®} | ----- | |

It can be seen that the compositions containing the non-ionic tensioactive agent permit a dissolution percentage to be obtained equivalent to the original commercial drug of ebastine, Ebastel^{®} 10 mg, without the need for prior micronisation of the ebastine.

## Claims

1. A solid pharmaceutical composition comprising at least a first and a second excipient mixed with a crystalline active principle in particles with a maximum particle size of less than 500 µm, of formula 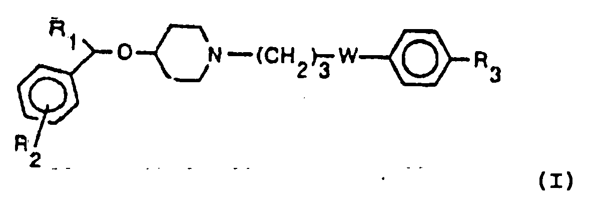 in which
- R1 is selected- from a group consisting of thienyl group, a phenyl group, a phenyl group substituted with a halogen, an alcoxy group with 1 to 6 carbon atoms or an alkyl group with 1 to 6 carbon atoms,
- R2 is selected from a group consisting of halogen atom, a hydrogen atom, an alcoxy group with 1 to 6 carbon atoms or an alkyl group with 1 to 6 carbon atoms,
- R3 is selected from a group consisting of halogen atom, a hydrogen atom, an alcoxy group with 1 to 6 carbon atoms, an alkyl group with 1 to 6 carbon atoms, an alkylthio group with 1 to 6 carbon atoms, a cycloalkyl group with 5 or 6 carbon atoms, or a group of formula: 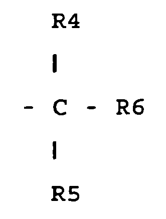
in which
R4 and R5 are, independently of each other, a hydrogen atom or an alkyl group with 1 to 6 carbon atoms,
R6 is a cycloalkyl group with 3 to 6 carbon atoms, or a hydroxymethyl, carboxy or alcoxycarbonyl group with 2 to 7 carbon atoms,
W is a carbonyl or hydroxymethylene group, or a pharmaceutically acceptable salt of said active principle,
**characterised in that** the first excipient is a non-ionic tensioactive agent.

2. A pharmaceutical composition according to claim 1, **characterised in that** it comprises between 1 and 25% by weight of the active principle.

3. A pharmaceutical composition according to claim 1 or 2, **characterised in that** it comprises between 8 and 12% by weight of the active principle.

4. A pharmaceutical composition according to claim 1, 2 or 3, **characterised in that** the active principle is ebastine.

5. A pharmaceutical composition according to one of claims 1 to 4, **characterised in that** the crystalline active principle has a mean particle size by volume of between 15 and 100 µm.

6. A pharmaceutical composition according to one of claims 1 to 5, **characterised in that** the crystalline active principle has a mean particle size by volume of between 16 and 80 µm.

7. A composition according to one of claims 1 to 6, **characterised in that**, by volume, the active principle has a granulometry in which 90% by volume of the particles have a particle size less than 225 µm.

8. A composition according to one of claims 1 to 6, **characterised in that**, by volume, the active principle has a granulometry in which 90% by volume of the particles have a particle size less than 150 µm.

9. A pharmaceutical composition according to claim 1, **characterised in that** the tensioactive agent is selected from among the group consisting of fatty esters of polyethoxylenated sorbitan.

10. A pharmaceutical composition according to claim 1, **characterised in that** the tensioactive agent is polyethoxylenated sorbitan monooleate.

11. A pharmaceutical composition according to one of claims 1 and 9 to 10, **characterised in that** it comprises between 0.1 and 10% by weight of the tensioactive agent.

12. A pharmaceutical composition according to one of claims 1 and 9 to 11, **characterised in that** it comprises between 0.5 and 2% by weight of the tensioactive agent.

13. A pharmaceutical composition according to claim 1, **characterised in that** the second excipient is a diluting agent.

14. A pharmaceutical composition according to claim 1, **characterised in that** the second excipient is a diluting agent selected from among the group consisting of lactose, lactose monohydrate, mannitol, cellulose, phosphates and inorganic carbonates and combinations thereof.

15. A pharmaceutical composition according to claim 1, **characterised in that** the second excipient is lactose monohydrate.

16. A pharmaceutical composition according to one of claims 11 to 15, **characterised in that** it comprises between 10 and 90% by weight of the second excipient.

17. A pharmaceutical composition according to one of claims 11 to 15, **characterised in that** it comprises between 50 and 70% by weight of the second excipient.

18. A pharmaceutical composition according to claim 1, **characterised in that** it furthermore comprises a binding agent.

19. A pharmaceutical composition according to claim 1, **characterised in that** it furthermore comprises a disintegrating agent.

20. A pharmaceutical composition according to claim 1, **characterised in that** it furthermore comprises a filling agent.

21. A pharmaceutical composition according to claim 1, **characterised in that** it furthermore comprises a lubricating agent.

22. A pharmaceutical composition according to any of the foregoing claims, **characterised in that** it is a compressed composition.

23. A pharmaceutical composition according to claim 22, **characterised in that** it comprises the crystalline active principle, a diluent, a binding agent, a disintegrating agent, a tensioactive agent and a compression coadjuvant agent.

24. A method for obtaining a pharmaceutical composition according to claim 1, **characterised in that** it comprises
preparing a first mixture comprising the crystalline active principle and a diluent,
passing the first mixture through a sieve with hole sizes between 0.5 and 0.7 mm, in order to obtain a first sieved mixture,
adding a disintegrating agent to the first sieved mixture in order to obtain a second mixture,
blending the second mixture with an aqueous solution of the tensioactive agent in order to obtain a blended mixture,
drying the blended mixture until a dry mixture is obtained,
passing the dry mixture through a sieve with hole sizes between 1.8 to 2.2 mm, in order to obtain a second sieved mixture,
mixing the second sieved mixture with a compression coadjuvant agent in order to obtain a compressible mixture, and
compressing at least part of the compressible mixture in order to obtain at least one tablet.

25. A method according to claim 24, **characterised in that** a binding agent is furthermore added to the second mixture.

26. A method according to claim 24 or 25, **characterised in that** a filling agent is furthermore added to the first mixture.

27. A method according to claim 24, 25 or 26, **characterised in that** the coadjuvant agent with which the second sieved mixture is mixed is a lubricating agent.

## Patentansprüche

1. Feste pharmazeutische Zusammensetzung, umfassend wenigstens ein erstes und ein zweites Excipiens, vermischt mit einem kristallinen Wirkstoff in Teilchen mit einer maximalen Teilchengröße von weniger als 500 µm, mit der Formel 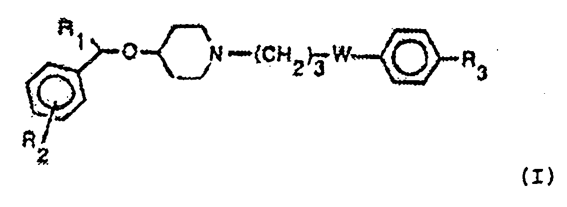 in welcher
- R₁ ausgewählt ist aus einer Gruppe bestehend aus einer Thienylgruppe, einer Phenylgruppe, einer Phenylgruppe substituiert mit einem Halogen, einer Alkoxygruppe mit 1 bis 6 Kohlenstoffatomen oder einer Alkylgruppe mit 1 bis 6 Kohlenstoffatomen,
- R₂ ausgewählt ist aus einer Gruppe bestehend aus einem Halogenatom, einem Wasserstoffatom, einer Alkoxygruppe mit 1 bis 6 Kohlenstoffatomen oder einer Alkylgruppe mit 1 bis 6 Kohlenstoffatomen,
- R₃ ausgewählt ist aus einer Gruppe bestehend aus einem Halogenatom, einem Wasserstoffatom, einer Alkoxygruppe mit 1 bis 6 Kohlenstoffatomen, einer Alkylgruppe mit 1 bis 6 Kohlenstoffatomen, einer Alkylthiogruppe mit 1 bis 6 Kohlenstoffatomen, einer Cycloalkylgruppe mit 5 oder 6 Kohlenstoffatomen oder einer Gruppe mit der Formel: 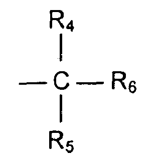
in welcher R₄ und R₅ unabhängig voneinander ein Wasserstoffatom oder eine Alkylgruppe mit 1 bis 6 Kohlenstoffatomen sind,
R₆ eine Cycloalkylgruppe mit 3 bis 6 Kohlenstoffatomen oder eine Hydroxymethyl-, Carboxy- oder Alkoxycarbonylgruppe mit 2 bis 7 Kohlenstoffatomen ist,
W eine Carbonyl- oder Hydroxymethylengruppe ist, oder einem pharmazeutisch annehmbaren Salz des Wirkstoffs,
**dadurch gekennzeichnet, dass** das erste Excipiens ein nicht-ionisches oberflächenaktives Mittel ist.

2. Pharmazeutische Zusammensetzung nach Anspruch 1, **dadurch gekennzeichnet, dass** sie zwischen 1 und 25 Gew.-% des Wirkstoffs umfasst.

3. Pharmazeutische Zusammensetzung nach Anspruch 1 oder 2, **dadurch gekennzeichnet, dass** sie zwischen 8 und 12 Gew.-% des Wirkstoffs umfasst.

4. Pharmazeutische Zusammensetzung nach Anspruch 1, 2 oder 3, **dadurch gekennzeichnet, dass** der Wirkstoff Ebastin ist.

5. Pharmazeutische Zusammensetzung nach einem der Ansprüche 1 bis 4, **dadurch gekennzeichnet, dass** der kristalline Wirkstoff eine volumengemittelte Teilchengröße zwischen 15 und 100 µm aufweist.

6. Pharmazeutische Zusammensetzung nach einem der Ansprüche 1 bis 5, **dadurch gekennzeichnet, dass** der kristalline Wirkstoff eine volumengemittelte Teilchengröße zwischen 16 und 80 µm aufweist.

7. Zusammensetzung nach einem der Ansprüche 1 bis 6, **dadurch gekennzeichnet, dass**, bezogen auf das Volumen, der Wirkstoff eine Granulometrie aufweist, in welcher 90 Vol.-% der Teilchen eine Teilchengröße von weniger als 225 µm aufweisen.

8. Zusammensetzung nach einem der Ansprüche 1 bis 6, **dadurch gekennzeichnet, dass**, bezogen auf das Volumen, der Wirkstoff eine Granulometrie aufweist, in welcher 90 Vol.-% der Teilchen eine Teilchengröße von weniger als 150 µm aufweisen.

9. Pharmazeutische Zusammensetzung nach Anspruch 1, **dadurch gekennzeichnet, dass** das oberflächenaktive Mittel ausgewählt ist aus der Gruppe bestehend aus Fettestern von polyethoxyliertem Sorbitan.

10. Pharmazeutische Zusammensetzung nach Anspruch 1, **dadurch gekennzeichnet, dass** das oberflächenaktive Mittel polyethoxyliertes Sorbitanmonooleat ist.

11. Pharmazeutische Zusammensetzung nach einem der Ansprüche 1 und 9 bis 10, **dadurch gekennzeichnet, dass** sie zwischen 0,1 und 10 Gew.-% des oberflächenaktiven Mittels umfasst.

12. Pharmazeutische Zusammensetzung nach einem der Ansprüche 1 und 9 bis 11, **dadurch gekennzeichnet, dass** sie zwischen 0,5 und 2 Gew.-% des oberflächenaktiven Mittels umfasst.

13. Pharmazeutische Zusammensetzung nach Anspruch 1, **dadurch gekennzeichnet, dass** das zweite Excipiens ein Verdünnungsmittel ist.

14. Pharmazeutische Zusammensetzung nach Anspruch 1, **dadurch gekennzeichnet, dass** das zweite Excipiens ein Verdünnungsmittel, ausgewählt aus der Gruppe bestehend aus Lactose, Lactosemonohydrat, Mannitol, Cellulose, Phosphaten und anorganischen Carbonaten und Kombinationen davon, ist.

15. Pharmazeutische Zusammensetzung nach Anspruch 1, **dadurch gekennzeichnet, dass** das zweite Excipiens Lactosemonohydrat ist.

16. Pharmazeutische Zusammensetzung nach einem der Ansprüche 11 bis 15, **dadurch gekennzeichnet, dass** sie zwischen 10 und 90 Gew.-% des zweiten Excipiens umfasst.

17. Pharmazeutische Zusammensetzung nach einem der Ansprüche 11 bis 15, **dadurch gekennzeichnet, dass** sie zwischen 50 und 70 Gew.-% des zweiten Excipiens umfasst.

18. Pharmazeutische Zusammensetzung nach Anspruch 1, **dadurch gekennzeichnet, dass** sie außerdem ein Bindemittel umfasst.

19. Pharmazeutische Zusammensetzung nach Anspruch 1, **dadurch gekennzeichnet, dass** sie außerdem ein Zerfallhilfsmittel umfasst.

20. Pharmazeutische Zusammensetzung nach Anspruch 1, **dadurch gekennzeichnet, dass** sie außerdem ein Füllmittel umfasst.

21. Pharmazeutische Zusammensetzung nach Anspruch 1, **dadurch gekennzeichnet, dass** sie außerdem ein Schmiermittel umfasst.

22. Pharmazeutische Zusammensetzung nach einem der vorangehenden Ansprüche, **dadurch gekennzeichnet, dass** sie eine verpresste Zusammensetzung ist.

23. Pharmazeutische Zusammensetzung nach Anspruch 22, **dadurch gekennzeichnet, dass** sie den kristallinen Wirkstoff, ein Verdünnungsmittel, ein Bindemittel, ein Zerfallhilfsmittel, ein oberflächenaktives Mittel und ein Presshilfsmittel umfasst.

24. Verfahren zum Erhalten einer pharmazeutischen Zusammensetzung nach Anspruch 1, **dadurch gekennzeichnet, dass** es umfasst
Herstellen eines ersten Gemisches, umfassend den kristallinen Wirkstoff und ein Verdünnungsmittel,
Leiten des ersten Gemisches durch ein Sieb mit Lochgrößen zwischen 0,5 und 0,7 mm, um ein erstes gesiebtes Gemisch zu erhalten,
Zugeben eines Zerfallhilfsmittels zu dem ersten gesiebten Gemisch, um ein zweites Gemisch zu erhalten,
Vermischen des zweiten Gemisches mit einer wässrigen Lösung des oberflächenaktiven Mittels, um ein vermischtes Gemisch zu erhalten,
Trocknen des vermischten Gemisches, bis ein trockenes Gemisch erhalten wird,
Leiten des trockenen Gemisches durch ein Sieb mit Lochgrößen zwischen 1,8 bis 2,2 mm, um ein zweites gesiebtes Gemisch zu erhalten,
Mischen des zweiten gesiebten Gemisches mit einem Presshilfsmittel, um ein verpressbares Gemisch zu erhalten, und
Verpressen wenigstens eines Teils des verpressbaren Gemisches, um wenigstens eine Tablette zu erhalten.

25. Verfahren nach Anspruch 24, **dadurch gekennzeichnet, dass** außerdem ein Bindemittel zu dem zweiten Gemisch zugegeben wird.

26. Verfahren nach Anspruch 24 oder 25, **dadurch gekennzeichnet, dass** außerdem ein Füllmittel zu dem ersten Gemisch zugegeben wird.

27. Verfahren nach Anspruch 24, 25 oder 26, **dadurch gekennzeichnet, dass** das Presshilfsmittel, mit dem das zweite gesiebte Gemisch gemischt wird, ein Schmiermittel ist.

## Revendications

1. Composition pharmaceutique solide comprenant au moins un premier et un second excipient mélangés à un principe actif cristallin en particules avec une dimension maximale de particules inférieure à 500 µm, de formule 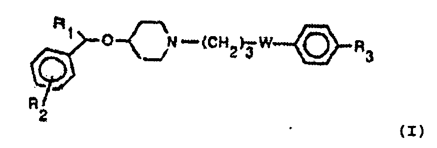 dans laquelle
- R1 est choisi dans le groupe constitué par un groupe thiényle, un groupe phényle, un groupe phényle substitué par un halogène, un groupe alcoxy avec 1 à 6 atomes de carbone ou un groupe alkyle avec 1 à 6 atomes de carbone,
- R2 est choisi dans le groupe constitué par un atome d'halogène, un atome d'hydrogène, un groupe alcoxy avec 1 à 6 atomes de carbone ou un groupe alkyle avec 1 à 6 atomes de carbone,
- R3 est choisi dans le groupe constitué par un atome d'halogène, un atome d'hydrogène, un groupe alcoxy avec 1 à 6 atomes de carbone, un groupe alkyle avec 1 à 6 atomes de carbone, un groupe alkylthio avec 1 à 6 atomes de carbone, un groupe cycloalkyle avec 5 ou 6 atomes de carbone, ou un groupe de formule : 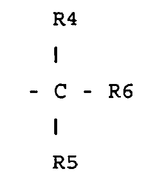
dans laquelle
R4 et R5 sont, indépendamment l'un de l'autre, un atome d'hydrogène ou un groupe alkyle avec 1 à 6 atomes de carbone,
R6 est un groupe cycloalkyle avec 3 à 6 atomes de carbone, ou un groupe hydroxyméthyle, carboxy ou alcoxycarbonyle avec 2 à 7 atomes de carbone,
W est un groupe carbonyle ou hydroxyméthylène, ou un sel pharmaceutiquement acceptable dudit principe actif,
**caractérisée en ce que** le premier excipient est un agent tensioactif non ionique.

2. Composition pharmaceutique selon la revendication 1, **caractérisée en ce qu'**elle comprend entre 1 et 25 % en masse de principe actif.

3. Composition pharmaceutique selon la revendication 1 ou 2, **caractérisé en ce que**'elle comprend entre 8 et 12 % en masse de principe actif.

4. Composition pharmaceutique selon la revendication 1, 2 ou 3, **caractérisée en ce que** le principe actif est l'ébastine.

5. Composition pharmaceutique selon l'une des revendications 1 à 4, **caractérisée en ce que** le principe actif cristallin a une dimension moyenne de particules en volume entre 15 et 100 µm.

6. Composition pharmaceutique selon l'une des revendications 1 à 5, **caractérisée en ce que** le principe actif cristallin a une dimension moyenne de particules en volume entre 16 et 80 µm.

7. Composition pharmaceutique selon l'une des revendications 1 à 6, **caractérisée en ce que**, en volume, le principe actif a une granulométrie dans laquelle 90 % en volume de particules ont une dimension moyenne de particules inférieure à 225 µm.

8. Composition pharmaceutique selon l'une des revendications 1 à 6, **caractérisée en ce que**, en volume, le principe actif a une granulométrie dans laquelle 90 % en volume de particules ont une dimension moyenne de particules inférieure à 150 µm.

9. Composition pharmaceutique selon la revendication 1, **caractérisée en ce que** l'agent tensioactif est choisi dans le groupe constitué par les esters gras de sorbitane polyéthoxyléné.

10. Composition pharmaceutique selon la revendication 1, **caractérisée en ce que** l'agent tensioactif est le monooléate de sorbitane polyéthoxyléné.

11. Composition pharmaceutique selon l'une des revendications 1 et 9 ou 10, **caractérisée en ce qu'**elle comprend entre 0,1 et 10 % en masse d'agent tensioactif.

12. Composition pharmaceutique selon l'une des revendications 1 et 9 à 11, **caractérisée en ce qu'**elle comprend entre 0,5 et 2 % en masse d'agent tensioactif.

13. Composition pharmaceutique selon la revendication 1, **caractérisée en ce que** le second excipient est un agent diluant.

14. Composition pharmaceutique selon la revendication 1, **caractérisée en ce que** le second excipient est un agent diluant choisi dans le groupe constitué par le lactose, le monohydrate de lactose, le mannitol, la cellulose, les phosphates et les carbonates minéraux et les combinaisons de ceux-ci.

15. Composition pharmaceutique selon la revendication 1, **caractérisée en ce que** le second excipient est le monohydrate de lactose.

16. Composition pharmaceutique selon l'une des revendications 11 à 15, **caractérisée en ce qu'**elle comprend entre 10 et 90 % en masse du second excipient.

17. Composition pharmaceutique selon l'une des revendications 11 à 15, **caractérisée en ce qu'**elle comprend entre 50 et 70 % en masse du second excipient.

18. Composition pharmaceutique selon la revendication 1, **caractérisée en ce qu'**elle comprend en outre un agent liant.

19. Composition pharmaceutique selon la revendication 1, **caractérisée en ce qu'**elle comprend en outre un agent délitant.

20. Composition pharmaceutique selon la revendication 1, **caractérisée en ce qu'**elle comprend en outre un agent de remplissage.

21. Composition pharmaceutique selon la revendication 1, **caractérisée en ce qu'**elle comprend en outre un agent lubrifiant.

22. Composition pharmaceutique selon l'une quelconque des revendications précédentes, **caractérisée en ce qu'**il s'agit d'une composition comprimée.

23. Composition pharmaceutique selon la revendication 22, **caractérisée en ce qu'**elle comprend le principe actif cristallin, un diluant, un agent liant, un agent délitant, un agent tensioactif et un agent coadjuvant de compression.

24. Procédé pour l'obtention d'une composition pharmaceutique selon la revendication 1, **caractérisée en ce qu'**elle comprend
la préparation d'un premier mélange comprenant le principe actif cristallin et un diluant,
le passage du premier mélange dans un tamis ayant des trous d'une dimension entre 0,5 et 0,7 mm, pour obtenir un premier mélange tamisé,
l'addition d'un agent délitant au premier mélange tamisé pour obtenir un second mélange,
la combinaison du second mélange avec une solution aqueuse de l'agent tensioactif, pour obtenir un mélange combiné,
le séchage du mélange combiné jusqu'à obtention d'un mélange sec,
le passage du mélange sec dans un tamis ayant des trous d'une dimension entre 1,8 et 2,2 mm, pour obtenir un second mélange tamisé,
le mélange du second mélange tamisé avec un agent coadjuvant de compression pour obtenir un mélange compressible et,
la compression d'au moins une partie du mélange compressible pour obtenir au moins un comprimé.

25. Procédé selon la revendication 24, **caractérisé en ce qu'**un agent liant est en outre ajouté au second mélange.

26. Procédé selon la revendication 24 ou 25, **caractérisé en ce qu'**un agent de remplissage est en outre ajouté au premier mélange.

27. Procédé selon la revendication 24, 25 ou 26, **caractérisé en ce que** l'agent coadjuvant mélangé avec le second mélange tamisé est un agent lubrifiant.
